# EUROPEAN PATENT SPECIFICATION

(11) **EP 3 267 873 B1**
(45) Date of publication and mention of the grant of the patent: **20.11.2019**
(21) Application number: 16713296.8
(22) Date of filing: 09.03.2016
(51) Int. Cl.: A61B 5/00, A43B 3/00, A43B 7/00, A63B 71/12, A61B 5/01

(54) **AN ARTICLE OF FOOTWEAR**
FUSSBEKLEIDUNGSARTIKEL
ARTICLE CHAUSSANT

(30) Priority: 09.03.2015 GB 201503928
(43) Date of publication of application: 17.01.2018
(73) Proprietor: Pearce, Christopher Jon, 278233 (SG); Curtin, David William, 249767 (SG); Curtin, William Anthony, Waterford City (IE)
(72) Inventor: PEARCE, Christopher Jon, 278233 (SG)
(74) Representative: O'Connor, Michael Donal
(86) International application number: PCT/EP2016/055064
(87) International publication number: WO 2016/142442

(56) References cited:
- EP-A2- 2 572 637
- US-A- 5 546 955
- US-A1- 2008 109 183
- NASIR MEHMOOD ET AL: "A flexible and low power telemetric sensing and monitoring system for chronic wound diagnostics", BIOMEDICAL ENGINEERING ONLINE, BIOMED CENTRAL LTD, LONDON, GB, vol. 14, no. 1, 1 March 2015 (2015-03-01), page 17, XP021214880, ISSN: 1475-925X, DOI: 10.1186/S12938-015-0011-Y

## Description

### Technical Field:

This invention relates to an article of footwear. More specifically, this invention relates to an article of footwear having a plurality of sensors for monitoring the physical properties of a patient's foot, and a system to enable the patient and/or a physician monitor the physical properties of the patient's foot.

### Background Art:

Diabetes Mellitus is a condition in which there is a disturbance in the metabolism of carbohydrate, fat and protein. Insulin is an important hormone in the regulation of metabolism and its function is affected in Diabetes Mellitus. There are two common types; Type I, or insulin dependent diabetes, typically affects younger patients and results from an inability of the individual to produce sufficient insulin. Type II, or non-insulin dependent diabetes affects older individuals and results from end-organ resistance to insulin. Type II diabetes is strongly associated with obesity and the incidence of this disease is rising to almost epidemic proportions in the world.

About 347 million people worldwide have diabetes according to recent World Health Organisation (WHO) statistics (http://www.who.int/features/factfiles/diabetes/facts/en/). It is likely that this is a significant under-estimate due to the fact that many patients do not realise that they have diabetes until a complication occurs. Diabetes is predicted to become the seventh leading cause of death in the world by the year 2030 and the total deaths from diabetes are projected to rise by more than 50% in the next 10 years.

Diabetes affects the eyes, kidneys, nerves, blood vessels and the body's response to infection. Diabetic foot complications such as infections, ulcers and Charcot arthropathy are the leading cause for hospital admission in the diabetic population. Diabetes is the commonest cause of limb amputation in the developed world. 30% of diabetic amputees will lose the other leg within 3 years and 60% die within 5 years of a limb amputation. Complications such as these are more common in Type II than Type I diabetes but Type II accounts for around 90% of all diabetes worldwide.

Although diabetes is a worldwide problem, it is instructive to consider the scale and development of the problem in a specific jurisdiction, in this case Singapore. In a 2004 survey, diabetes was shown to affect 8.2% of the population in Singapore with this figure rising to 16.7% of people in their 5th decade and 28.7% of people in their 6th decade of life (source: Ministry of Health, Singapore). Furthermore, Type II diabetes mellitus is a growing problem in Singapore, with 11.3% of adults affected with the disease in 2010. It is estimated that up to 2.9% of these patients may be affected by Charcot's arthropathy (Leung HC, Ho YC, Wong WC. Charcot foot in a Hong Kong Chinese diabetic population. Hong Kong Medical Journal 2009; 15: 191-5.). Charcot's arthropathy is a disease affecting the bones, joints and proprioception of the foot, resulting in changes in the structure of the foot and its weight-bearing areas, predisposing to abnormal callus development, ulcer formation and predisposition for deep infection. The joints are destroyed due to overloading resulting from a lack of protective reflexes in the insensate foot. It was originally described in syphilis but Diabetes is now by far the leading cause. Clinically, in the acute phase of the disease, the affected joint becomes hot and swollen. Later, if allowed to progress without treatment, the joint collapses leading to the limb becoming misshapen and loss of function. Pressure areas in the insensate foot can quickly lead to skin ulcers, especially if the foot is misshapen and therefore more likely to rub on footwear.

Diabetes also affects the blood vessels and the immune system which together mean that these ulcers are very slow to heal and very readily become infected. Once infected, treatment is difficult, for the same reasons mentioned above, and amputation is all too often necessary. As a result, the five year major amputation rates in Charcot's have been found to be as high as 11% (Moulik PK, Mtonga R, Gill GV. Amputation and mortality in new-onset diabetic foot ulcers stratified by etiology. Diabetes Care 2003; 26: 461-4.). Diabetics have more than ten times the risk of non-diabetics for lower limb amputations, and account for more than half the number of non-traumatic lower limb amputations (Icks A et al. Incidence of lower-limb amputations in the diabetic compared to the non-diabetic population. Findings from nationwide insurance data, Germany, 2005-2007. Experimental and Clinical Endocrinology and Diabetes 2009; 117:500-4).

The World Health Organisation (WHO) guidelines on Non-Communicable Diseases in 2010 recognised this growing trend worldwide in both increasing numbers of diabetics and the health and economic burden generated by diabetics who needed lower limb amputations. In their strategy for individual interventions to improve health care in diabetics, they recommended foot care for those at risk of developing foot ulcers. The mainstay of treatment of the diabetic foot is with the use of boots or casts which even out the pressure over the foot and restrict the patients from bearing weight on the limb. The patient will typically be reviewed in the clinic at the hospital every 1-2 weeks. Assessment consists of clinical evaluation and the measurement of the skin temperature between the affected and the unaffected leg. In the treatment of a Charcot Arthropathy the patient's foot will be kept immobilised in a boot or cast until the difference in skin temperature is within 2 degrees Celsius. Compliance with treatment is vital however it is often lacking in these patients especially if they are not well motivated and do not understand the treatment.

At present, there is currently no suitable device to monitor parameters in the diabetic foot, such as skin temperature and pressure. These parameters have a direct effect on management as well as prognosis of the disease (Moura-Neto A et al. Charcot foot: skin temperature as a good clinical parameter for predicting disease outcome. Diabetes Research and Clinical Practice 2012; 96(2): e11-4).

Various solutions to one or more aspects of the problem have been proposed. For example, DE102011012458 (in the name of Moticon GmbH) discloses a pressure measuring sole for use by a patient with diabetes to monitor the pressure distribution in the foot sole of the patient. US5642096 (in the name of Paromed Medizintechnik GmbH) discloses a device for the prevention of ulcers in the feet of diabetes patients that detects both the pressure and temperature values to which the feet are exposed. This device comprises a sensor in a hydrocell carried in the shoe inner sole which monitors changes in the temperature of the hydrocell.

US2013021181 (in the name of Medhab, LLC) describes a sensor system for monitoring a foot during treatment and rehabilitation. The device comprises a temperature sensor array having a plurality of sensors in the form of an insole for detecting the temperature of a patient's diabetic foot at a plurality of locations about the sole of the foot.

US2011/0015498 (in the name of Commonwealth Scientific and Industrial Research OR) discloses a sock having a number of pressure sensors on the sole of the sock and a temperature sensor for monitoring a patient's diabetic foot at a plurality of locations about the sole of the foot.

US 5 546 955 A discloses a temperature monitoring sock provided with multiple sensors on the lower leg portion.

It is an object of the present invention to provide an article of footwear that overcomes at least some of the above-identified problems. It is a further object of the present invention to provide a useful choice to the consumer.

### Summary of Invention:

According to the invention there is provided an article of footwear for monitoring the physical properties of a patient's diabetic foot comprising a plurality of temperature sensors each of which is operable to measure skin temperature adjacent the sensor, and in which one of the sensors comprises a reference sensor located remote from the other sensors, the reference sensor being arranged for location on the patient's lower leg above and spaced apart from the ankle.

By having such an article of footwear, it will be possible to measure the actual skin temperature at a plurality of points adjacent the sensors around the patient's foot. This will give a far better indication of the patient's condition and will help in the treatment and management of the condition. The article of footwear will ultimately improve health and decrease morbidity related to diabetes mellitus and Charcot's foot. More specifically, by having a reference sensor located remote from the other sensors in this location, it will be possible to detect whether there has been a change in the difference in temperature between one or more infected areas and a healthy area in which the reference sensor is located. This will enable patients and physicians to monitor the efficacy of the treatment and will alert them to any improvement or deterioration of the patient's condition.

In one example there is provided an article of footwear in which the reference sensor is mounted on a carrier physically separate from the remainder of the article of footwear. By having the reference sensor on a carrier physically separate from the remainder of the article of footwear, the reference sensor may be mounted on the patient's other leg thereby providing a very good reference point for the physician.

In one embodiment of the invention there is provided an article of footwear in which there is provided at least one temperature sensor located at an ankle region of the article of footwear.

In one embodiment of the invention there is provided an article of footwear in which there is provided a temperature sensor located at an inner ankle region and a temperature sensor located at an outer ankle region of the article of footwear. Preferably, the temperature sensors will be located at or adjacent to the malleolus.

In one embodiment of the invention there is provided an article of footwear in which there is provided at least one temperature sensor located at a sole region of the article of footwear.

In one embodiment of the invention there is provided an article of footwear in which there is provided a temperature sensor located at a heel region of the sole of the article of footwear, a temperature sensor located in the region of a 1^{st} metatarsal head region of the sole of the article of footwear, and a temperature sensor located in the region of a 5^{th} metatarsal head region of the sole of the article of footwear.

In one embodiment of the invention there is provided an article of footwear in which there is provided a pitch sensor mounted on the article of footwear. This is seen as a preferred example. By providing a pitch sensor, it will be possible to detect whether or not the article of footwear has been in an elevated position as perhaps prescribed by the physician and if so, for how long the article of footwear has been in the elevated position. This will enable the patient and physician to monitor the patient's compliance with the recommended treatment.

In one example there is provided an article of footwear in which the pitch sensor comprises an accelerometer.

In one embodiment of the invention there is provided an article of footwear in which there is provided a pressure sensor mounted on the article of footwear. In another embodiment, there are provided a pair of pressure sensors mounted on the article of footwear. Ideally, one of the pair of pressure sensors will be located on the sole adjacent the heel and the other of the pair of pressure sensors will be located on the sole adjacent the toes.

In one embodiment of the invention there is provided an article of footwear in which the pressure sensor is located at the sole region of the article of footwear.

In one embodiment of the invention there is provided an article of footwear in which there is provided an activity sensor to detect when the boot is in use on a patient's foot.

In one embodiment of the invention there is provided an article of footwear in which there is provided a glucose sensor mounted on the article of footwear.

In one embodiment of the invention there is provided an article of footwear in which there is provided a sock and in which one or more of the sensors are attached to the sock. This is seen as a simple way of mounting the sensors on the article of footwear that will enable the sensors to be retrospectively fitted to several of the leading brands of boots used in the treatment of diabetic foot.

In one embodiment of the invention there is provided an article of footwear in which the article of footwear comprises a microprocessor for collating data from each of the sensors and a communications module for transmitting the collated data to a remote monitoring station.

In one embodiment of the invention there is provided a system for monitoring the physical properties of a patient's foot comprising an article of footwear and a remote computing device running computer program code capable of receiving data from a communications module and displaying the data on a user interface of the computing device. In this way, the data from each of the sensors may be transmitted to a remote monitoring station for display and analysis by the patient or a physician.

In one embodiment of the invention there is provided a system for monitoring the physical properties of a patient's foot in which the computing device is a smartphone operated by a patient. This is seen as a particularly preferred embodiment of the present invention. By providing the data to a smartphone of the patient, the patient will be engaged more intimately in their own care and they can see from the data whether their actions are having a positive or negative effect on their condition. If their behavior is having a negative effect on their condition, they can adjust their behavior accordingly to improve their prognosis. If their behavior is having a positive effect on their condition, the data provides them with encouragement that their actions are having the desired effect.

In one embodiment of the invention there is provided a system for monitoring the physical properties of a patient's foot in which the computing device is operated by a physician. This is also seen as a particularly preferred embodiment of the present invention. By providing the physician with the data, a physician in a remote location can treat their patients remotely and will be able to identify whether a patient needs to come in for a visit or whether the visit can be postponed. This will assist with alleviating otherwise stretched resources in the health services.

In one embodiment of the invention there is provided a system of footwear in which the article of footwear communicates with the computing device wirelessly.

### Brief Description of the Drawings:

The invention will now be more clearly understood from the following description of some embodiments thereof given by way of example only with reference to the accompanying drawings, in which:-
Figure 1 is a perspective view of an article of footwear according to the invention;
Figure 2 is a diagrammatic representation of the placement of temperature sensors at a sole region of a patient's foot;
Figure 3 is a diagrammatic representation of the placement of temperature sensors at an ankle region of a patient's foot;
Figure 4 is a diagrammatic representation of the placement of temperature sensors at an ankle region of a patient's foot and a reference temperature sensor;
Figure 5 is a diagrammatic representation of a system in which the article of footwear of Figure 1 may be used; and
Figure 6 is a view similar to Figure 1 showing an alternative arrangement of sensors.

### Detailed Description of the Drawings:

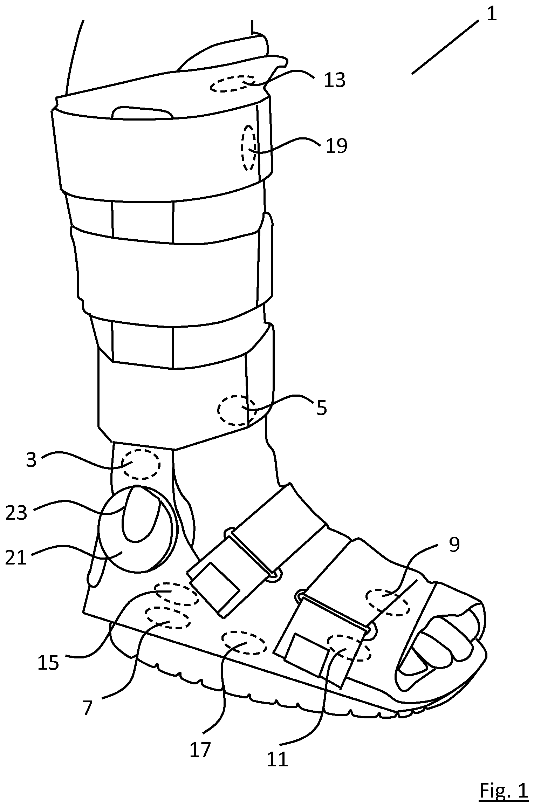

Referring to Figure 1, there is shown an article of footwear, indicated generally by the reference numeral 1, in this case a surgical boot. The article of footwear comprises a plurality of temperature sensors 3, 5, 7, 9, 11 and 13, shown here in dashed outline, operable to measure the skin temperature adjacent the sensor. The article of footwear further comprises a pressure sensor 15, a pitch sensor 17 and an activity sensor 19. In addition to the foregoing, the article of footwear comprises a microprocessor 21 and a wireless communications module 23.

In use, the temperature sensors 3, 5, 7, 9, 11 and 13 are operable to measure the skin temperature of the patient's foot adjacent the sensor. Sensor 3 is located at an outer ankle region of the foot and sensor 5 is located at an inner ankle region of the foot. The sensors 3, 5 are both located at or adjacent to the patient's malleolus. Temperature sensors 7, 9 and 11 are located at a sole region of the foot. More specifically, sensor 7 is located at the heel of the sole region of the foot, sensor 9 is located at the 1^{st} metatarsal head region of the sole of the article of footwear, and the temperature sensor 11 located in the region of a 5^{th} metatarsal head region of the sole of the article of footwear. Temperature sensor 13 is a reference sensor and is located up the leg of the patient remote from the foot of the patient. The temperature reference sensor 13 allows for more accurate assessment of the temperature of the affected foot/ankle by correcting for ambient temperature and the patient's body temperature. Importantly, it is the difference in the temperature of the affected joint compared to the other healthy joint that determines the length of treatment in the boot required in Charcot arthropathy. Typically, when the affected side is within 2 degrees Celsius of the other side, the acute phase of Charcot arthropathy is considered to be over and the cast or boot may be removed and the patient may be allowed to bear weight on the limb again. Each of the temperature sensors sends it's readings to the microprocessor 21 through wired or wireless communications.

At the same time, pressure sensor 15 detects the amount of pressure the patient is exerting on the ground (or that the ground is exerting on the limb) and sends that data to the microprocessor 21. Pitch sensor 17, provided by way of an accelerometer, detects the orientation of the boot, i.e. whether or not the boot is raised or lowered, and sends that data to the microprocessor 21. Finally, sensor 19 detects whether or not the boot is in use at that moment in time or whether the boot is not currently being worn by the patient and sends that information to the microprocessor 21. The microprocessor 21, having received the relevant data, can then cause the wireless communications module 23 to transmit the data to a remote location, such as a computing device operated by the patient and/or the physician.

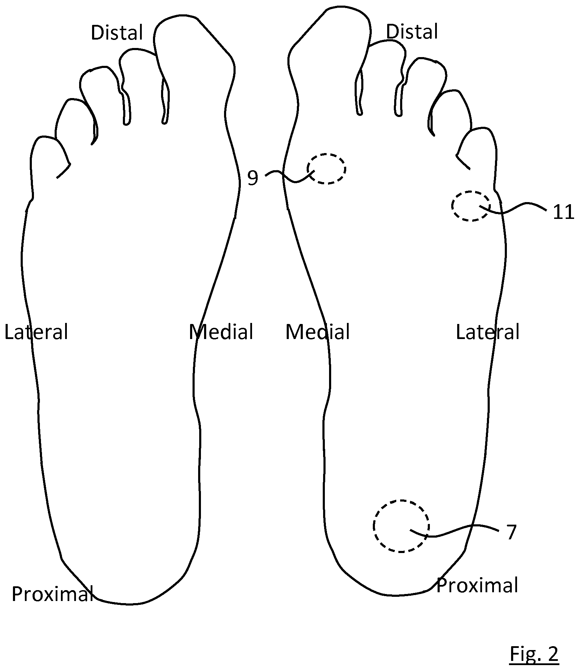

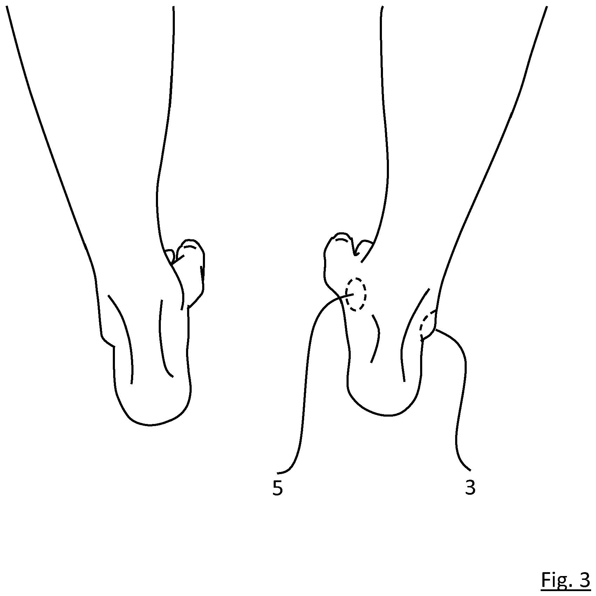

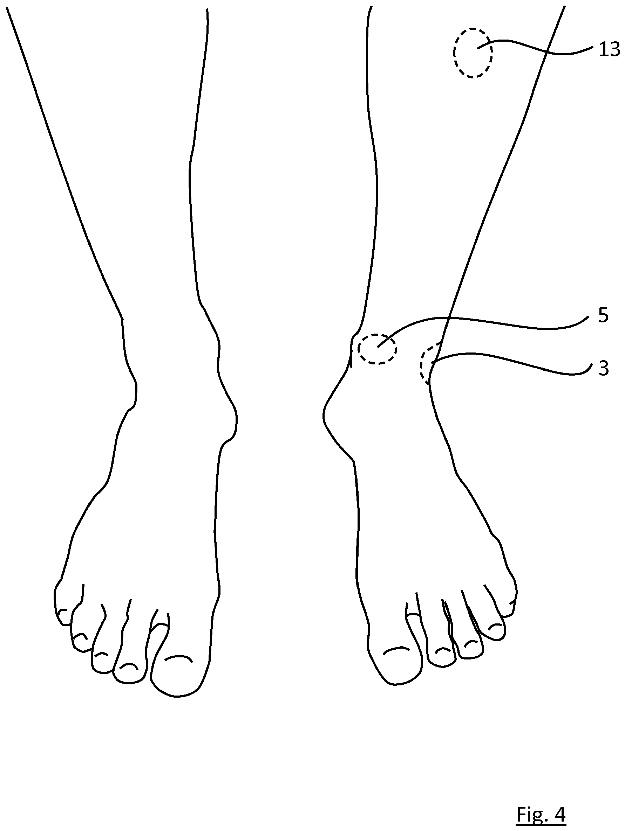

Referring now to Figures 2 to 4 inclusive, there is shown diagrammatically where on the patient's foot and leg that the temperature sensors 3, 5, 7, 9, 11 and 13 are located. Referring specifically to Figure 2, the preferred location for the temperature sensors 7, 9, and 11 for detecting the temperature of a plurality of points on the sole of the patient's foot are shown. Temperature sensor 7 is located at the heel area of the patient's foot, temperature sensor 9 is located at the 1^{st} metatarsal head region of the sole of the patient's foot, and the temperature sensor 11 located in the region of a 5^{th} metatarsal head region of the sole of the patient's foot. Referring specifically to Figure 3, the temperature sensor 3 is located at an outer ankle region of the foot at the malleolus and the temperature sensor 5 is located at an inner ankle region of the foot, again at the malleolus. Referring now specifically to Figure 4, there is shown the position of the reference temperature sensor 13 located up the lower leg remote from the foot and from the other sensor locations (only two of which, location of sensors 3 and 5, are shown).

It will be understood that more or less sensors that those shown in Figures 1 to 4 could be provided. For example, it is envisaged that a further temperature sensor may be placed over the dorsum of the midfoot. This will be advantageous for patients with midfoot Charcot. Indeed, the reference temperature sensor 13 may be located on the patient's other leg instead of on the same leg as the temperature sensors 3, 5, 7, 9, and 11. The sensors can communicate with the microprocessor 21 through wireless communications. For example, a WPAN such as a ZigBee ® network could be used to good effect to allow communications between the sensors and the microprocessor. The microprocessor 21 thereafter uses another wireless communications module 23 to communicate the data to a remote location (not shown). The wireless communications module may use Bluetooth ® or other wireless communications to transmit the data to the remote location. Bluetooth ® is seen as useful due to its compatibility with smartphones and other devices.

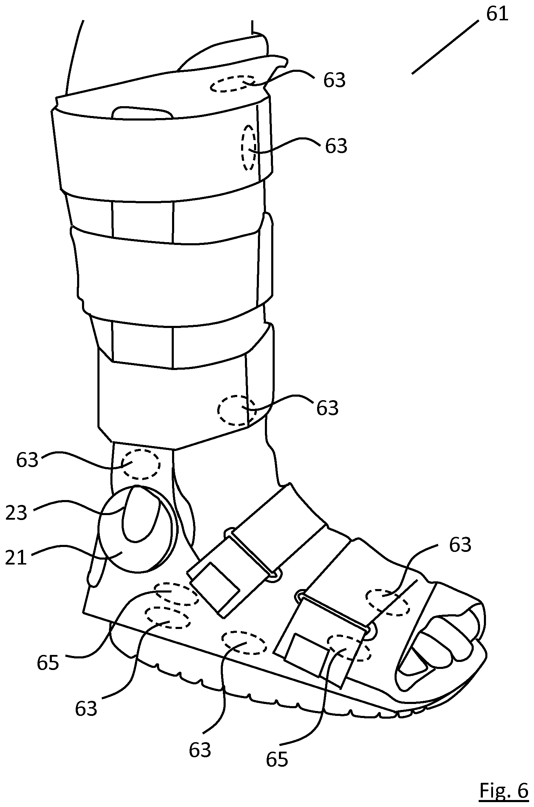

Referring now to Figure 6, there is shown a view similar to Figure 1 showing an alternative embodiment of the article of footwear according to the invention, indicated generally by the reference numeral 61, where like parts have been given the same reference numeral as before. The article of footwear 61 comprises a plurality of temperature sensors 63 and a pair of pressure sensors 65. One of the pair of pressure sensors 65 is located adjacent the heel of the patient's diabetic foot and the other of the pair of pressure sensors 65 is located adjacent the toes of the patient's diabetic foot. This will help to detect if the patient is putting more weight on the heel or the toes of the foot or if the weight is evenly distributed which can assist in the treatment of the patient and the identification of potential problems. In the embodiment shown, either or both of the two temperature sensors located remote from the patient's foot adjacent the top of the article of footwear can be used as the reference sensor.

It is envisaged that the reference sensor 13 will be spaced as far apart from the other temperature sensors monitoring the temperature of the patient's foot and/or ankle. Preferably, the reference sensor will be located up along the leg more than 0.2 metres above the sole of the patient's foot (when the patient is in an upright position). More preferably, the reference sensor will be located up along the leg more than 0.25 metres above the sole of the patient's foot. Ideally, the temperature sensor will be located equal to or more than 0.3 metres above the sole of the patient's foot. By spacing the reference sensor from the other sensors in this location, a more accurate assessment of the patient's condition is possible and a more effective treatment regime can be implemented.

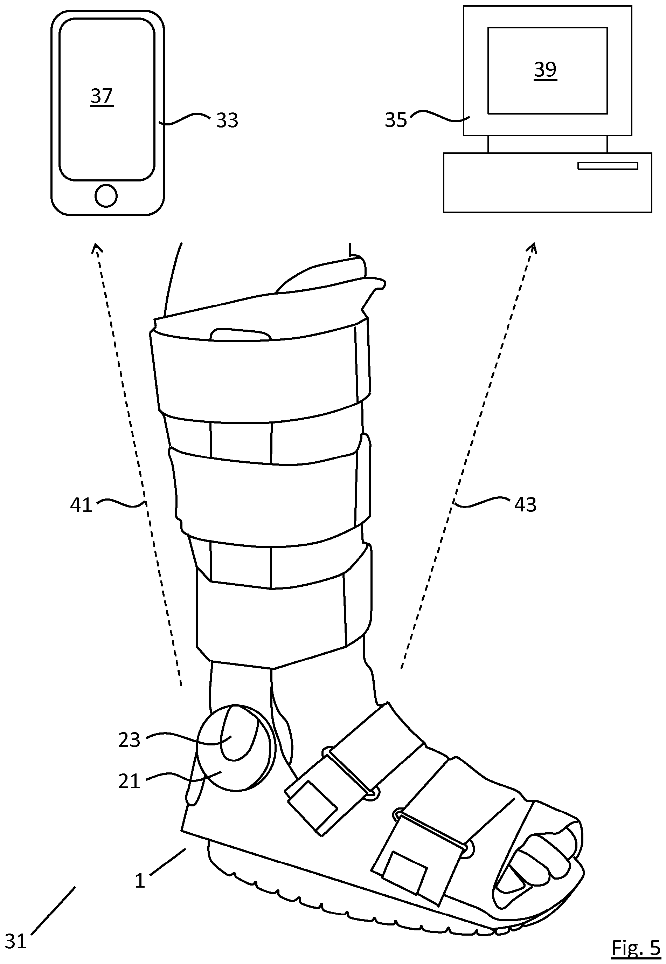

Referring to Figure 5, there is shown a diagrammatic representation of a system in which the article of footwear 1 may be used, indicated generally by the reference numeral 31. The system 31 comprises the boot 1, a smartphone 33 and another computing device, in this case a personal computer (PC) 35. The smartphone 33 has a user interface 37 and may be one operated by a physician, the patient themselves or a carer of the patient. The PC 35 also has a user interface 39 and may be one operated by the patient or more preferably by a physician located remotely from the patient. The wireless communications module 23 of the boot 1 can communicate with the smartphone 33 over communication channel 41 and can communicate with the PC 35 over communication channel 43.

In use, the microprocessor 21 on the article of footwear gathers data from the sensors thereon and the communications module 23 transmits the data to the smartphone 33. Ideally, the smartphone 33 will have software loaded thereon (an "App") that will receive the data and display the data in a meaningful manner on the user interface 37 of the smartphone 33. In this way, if the patient is in possession of the smartphone, they can monitor their own progress easily. The smartphone software may also have the ability to forward that data onwards from the smartphone 33 to the remote PC 35, which is preferably located in a hospital or other medical facility where it is monitored by a physician. The data from the sensors, sent via the smartphone 33, is then displayed on the user interface 39 of the PC 35. In this way, the physician can monitor the progress and the compliance of the patient with the recommended treatment (i.e. rest periods, elevation of the foot, keeping pressure off the foot, wearing the boot for certain periods of time each day and the like). It will be understood that the communications module 23 may be configured to communicate directly with the PC 35 however this is not preferred as this will require a more power hungry communications module 23.

It will be understood from the foregoing that it is proposed to provide an intelligent boot which can electronically monitor the parameters in a patient with diabetic foot conditions including Charcot's arthropathy, in order to provide feedback for both the patient and the doctor. The parameters preferably include skin temperature, pressure, elevation and compliance. This data is collected and stored on a microprocessor 21, which will then be transferred remotely, to the patients' smartphone 33 and to hospital IT systems 35 so that the patients' progress can be monitored by the physicians attending to the patient as well as the patients themselves. The data is uploaded to both the smartphone 33 and the PC 35 where a software package will collate the results and present them in tabular and/or graphic form that is easy for the patient in particular to understand and which allows them to track their progress. There are many advantages to collecting this data. It is envisaged that compliance with the treatment will improve, once patients can see, in graphic form, the results of their lifestyle modifications. The physician or podiatrist can also review these graphs to decide on which areas to focus on and how to adequately counsel patients on their review visits. Additionally, remote data transfer, possibly via a WiFi ® connection, will allow physicians to monitor their patients from afar, thus potentially reducing the number of follow up appointments required, as well as highlighting which patients should be given earlier reviews. This will potentially decrease the financial burden on both the individual patient and the taxpayer, as truly individualized treatment can be given.

Throughout the specification, the invention has been described in terms of a boot. However, it will be understood that the invention is not limited to the boot as shown (unless otherwise so limited in the claims) and other articles of footwear could be used to equally good effect. It is envisaged that the article of footwear could also be provided in the form of a sock. The sock is advantageous as it may be used in conjunction with a number of different and indeed pre-existing boots thereby reducing the spend required and deriving more benefit from existing equipment. Furthermore, the sock can be used on either foot and therefore only one device may have to be provided. The sock could be used to measure the qualities of the diabetic foot and then interchanged to the other foot to monitor the qualities of the patient's healthy foot so that a useful comparison can be drawn between the healthy foot and the unhealthy foot.

The sensors 3, 5, 7, 9, 11, 13, 15, 17 and 19 in Figure 1 are shown in dashed outline as they will be located out of view inside the boot in proximity to the patient's foot/leg. The sensors may be mounted in a sock 25 of the boot. The sock 25 is typically interchangeable in the boot in any event and this will allow the sensors to be retrofitted to an existing article of footwear 1. It is proposed to design modifications which can be fitted easily to an existing model of boot used in the treatment of diabetic foot complications. These would include temperature sensors to be placed within the boot or sewn into the sock at common sites of ulceration. It is, however vitally important that these sensors do not apply any pressure to the skin.

In the embodiment described above, in addition to the temperature sensors 3, 5, 7, 9, 11 and 13, a sensor 15 is placed over the heel which can detect whether or not the patient has been putting any weight through the limb or not. There is also a sensor 17 which records the pitch of the boot so that it is possible to tell for how many hours of the day the patient is keeping the foot elevated. There is also a sensor 19 monitoring to see for how many hours of the day the patient has worn the boot. It will be understood that the output from other sensors such as the temperature sensors 3, 5, 7, 9, 11, and 13, the pressure sensor 15 or the pitch sensor 17 could be used to detect whether the patient is wearing the boot and a separate sensor 19 for this task may not be required. The size and weight of the components should be kept to a minimum and they should be housed in sturdy, waterproof and shock resistant casings where possible.

It will be understood that parts of the system and method according to the present invention will be implemented/performed largely in software and therefore the present invention extends also to computer programs, on or in a carrier, comprising program instructions for causing a computer or a smartphone to carry out one or more steps of the method. The computer program may be in source code format, object code format or a format intermediate source code and object code. The computer program may be stored on or in a carrier, in other words a computer program product, including any computer readable medium, including but not limited to a floppy disc, a CD, a DVD, a memory stick, a tape, a RAM, a ROM, a PROM, an EPROM or a hardware circuit. The computer program product may be stored in the memory of a computing device and the present invention is intended to extend to computing devices programmed to implement the method according to the present invention.

It will be further understood that the present invention may be performed on two, three or more machines or components with certain parts of the computer-implemented method being performed by one machine or component and other parts of the computer-implemented method being performed by another machine or component. The devices may be part of a LAN, WLAN or could be connected together over a communications network including but not limited to the internet. One or more of the method steps could be performed "in the cloud", meaning that remotely located processing power may be utilised to process certain method steps of the present invention. Accordingly, it will be understood that many of the method steps may be performed remotely, by which it is meant that the method steps could be performed either on a separate machine in the same locality or jurisdiction or indeed on a separate machine or machines in one or several remote jurisdictions. For example, the PC, smartphone and boot may be in one jurisdiction or located in different jurisdictions. The present invention and claims are intended to also cover those instances where the method is performed across two or more machines or pieces of apparatus located in one or more jurisdictions and those situations where the parts of the system are spread out over one or more jurisdictions.

In this specification the terms "include, includes, included and including" and the terms "comprise, comprises, comprised and comprising" are all deemed totally interchangeable and should be afforded the widest possible interpretation.

Several embodiments and examples are disclosed in the present text. The scope of protection, however, is solely defined by the wording of the appended claims.

## Claims

1. An article of footwear (1) for monitoring the physical properties of a patient's diabetic foot comprising a plurality of temperature sensors (3, 5, 7, 9, 11, 13) each of which is operable to measure skin temperature adjacent the sensor, including a temperature sensor (3, 5) located at an ankle region of the article of footwear, and in which one of the sensors (13) comprises a reference sensor located remote from the other sensors, the reference sensor (13) being arranged for location on the patient's lower leg above and spaced apart from the ankle.

2. An article of footwear (1) as claimed in claim 1 in which the reference sensor is mounted on a carrier physically separate from the remainder of the article of footwear.

3. An article of footwear (1) as claimed in claim 1 or 2 in which the temperature sensor located at an ankle region of the article of footwear is located at or adjacent to the malleolus.

4. An article of footwear (1) as claimed in any preceding claim in which there is provided a temperature sensor located at an inner ankle region and a temperature sensor located at an outer ankle region of the article of footwear.

5. An article of footwear (1) as claimed in any preceding claim in which there is provided at least one temperature sensor (7, 9, 11) located at a sole region of the article of footwear.

6. An article of footwear (1) as claimed in claim 5 in which there is provided a temperature sensor (7) located at a heel region of the sole of the article of footwear, a temperature sensor (9) located in the region of a 1^{st} metatarsal head region of the sole of the article of footwear, and a temperature sensor (11) located in the region of a 5^{th} metatarsal head region of the sole of the article of footwear.

7. An article of footwear (1) as claimed in any preceding claim in which there is provided a pitch sensor (17) mounted on the article of footwear.

8. An article of footwear (1) as claimed in any preceding claim in which there is provided a pressure sensor (15, 65) mounted on the article of footwear.

9. An article of footwear (1) as claimed in claim 8 in which there are provided a pair of pressure sensors (65) located at the sole region of the article of footwear, one of which is located adjacent the heel of the article of footwear and the other of which is located adjacent the toe of the article of footwear.

10. An article of footwear (1) as claimed in any preceding claim in which there is provided an activity sensor (19) to detect when the boot is in use on a patient's foot.

11. An article of footwear (1) as claimed in any preceding claim in which there is provided a glucose sensor mounted on the article of footwear.

12. An article of footwear (1) as claimed in any preceding claim in which the reference sensor (13) is arranged for location on the patient's lower leg up along the leg more than 0.20 metres above the sole of the patient's foot.

13. A system for monitoring the physical properties of a patient's foot comprising:
an article of footwear (1) as in claim 1,
and in which the article of footwear comprises a microprocessor for collating data from each of the sensors and a communications module for transmitting the collated data to a remote monitoring station; and
a remote computing device running computer program code capable of receiving data from the communications module and displaying the data on a user interface of the computing device.

14. A system for monitoring the physical properties of a patient's foot as claimed in claim 13 in which the computing device is a smartphone operated by a patient.

15. A system for monitoring the physical properties of a patient's foot as claimed in claim 13 in which the computing device is operated by a physician.

## Patentansprüche

1. Fußbekleidungsartikel (1) zum Überwachen der physikalischen Eigenschaften eines diabetischen Fußes eines Patienten, umfassend eine Vielzahl von Temperatursensoren (3, 5, 7, 9, 11, 13), die jeweils wirksam sind, um die Hauttemperatur dem Sensor benachbart zu messen, umfassend einen in einem Knöchelbereich des Fußbekleidungsartikels liegenden Temperatursensor (3, 5), und wobei einer der Sensoren (13) einen entfernt von den anderen Sensoren liegenden Bezugssensor umfasst, wobei der Bezugssensor (13) zum Platzieren am Unterschenkel des Patienten oberhalb und in einem Abstand von dem Knöchel angeordnet ist.

2. Fußbekleidungsartikel (1) nach Anspruch 1, wobei der Bezugssensor an einem Träger angebracht ist, der physisch von dem Rest des Fußbekleidungsartikels getrennt angebracht ist.

3. Fußbekleidungsartikel (1) nach Anspruch 1 oder 2, wobei sich der in einem Knöchelbereich des Fußbekleidungsartikels liegende Temperatursensor an dem Fußknöchel oder diesem benachbart liegt.

4. Fußbekleidungsartikel (1) nach einem der vorangehenden Ansprüche, wobei ein in einem Innenknöchelbereich liegender Temperatursensor und ein in einem Außenknöchelbereich des Fußbekleidungsartikels liegender Temperatursensor vorgesehen sind.

5. Fußbekleidungsartikel (1) nach einem der vorangehenden Ansprüche, wobei mindestens ein in einem Sohlenbereich des Fußbekleidungsartikels liegender Temperatursensor (7, 9, 11) vorgesehen ist.

6. Fußbekleidungsartikel (1) nach Anspruch 5, wobei ein in einem Fersenbereich der Sohle des Fußbekleidungsartikels liegender Temperatursensor (7), ein in dem Bereich eines ersten Mittelfußköpfchenbereichs der Sohle des Fußbekleidungsartikels liegender Temperatursensor (9) und ein im Bereich eines fünften Mittelfußköpfchenbereichs der Sohle des Fußbekleidungsartikels liegender Temperatursensor (11) vorgesehen sind.

7. Fußbekleidungsartikel (1) nach einem der vorangehenden Ansprüche, wobei ein an dem Fußbekleidungsartikel angebrachter Neigungssensor (17) vorgesehen ist.

8. Fußbekleidungsartikel (1) nach einem der vorangehenden Ansprüche, wobei ein an dem Fußbekleidungsartikel angebrachter Drucksensor (15, 65) vorgesehen ist.

9. Fußbekleidungsartikel (1) nach Anspruch 8, wobei ein Paar in dem Sohlenbereich des Fußbekleidungsartikels liegende Drucksensoren (65) vorgesehen ist, von denen einer der Ferse des Fußbekleidungsartikels benachbart liegt und der andere dem Zeh des Fußbekleidungsartikels benachbart liegt.

10. Fußbekleidungsartikel (1) nach einem der vorangehenden Ansprüche, wobei ein Aktivitätssensor (19) vorgesehen ist, um zu erkennen, wann sich der Stiefel am Fuß eines Patienten in Gebrauch befindet.

11. Fußbekleidungsartikel (1) nach einem der vorangehenden Ansprüche, wobei ein an dem Fußbekleidungsartikel angebrachter Glukosesensor vorgesehen ist.

12. Fußbekleidungsartikel (1) nach einem der vorangehenden Ansprüche, wobei der Bezugssensor (13) zum Platzieren am Unterschenkel des Patienten mehr als 0,20 m oberhalb der Sohle des Fußes des Patienten am Bein entlang nach oben angeordnet ist.

13. System zum Überwachen der physikalischen Eigenschaften eines Fußes eines Patienten, das Folgendes umfasst:
einen Fußbekleidungsartikel (1) nach Anspruch 1, wobei der Fußbekleidungsartikel einen Mikroprozessor zum Zusammentragen von Daten von jedem der Sensoren und ein Kommunikationsmodul zum Übermitteln der zusammengetragenen Daten an eine entfernte Überwachungsstation umfasst; und
eine Computerprogrammcode ausführende entfernte Rechenvorrichtung, die in der Lage ist, Daten von dem Kommunikationsmodul zu empfangen und die Daten auf einer Benutzeroberfläche der Rechenvorrichtung anzuzeigen.

14. System zum Überwachen der physikalischen Eigenschaften eines Fußes eines Patienten nach Anspruch 13, wobei es sich bei der Rechenvorrichtung um ein von einem Patienten bedientes Smartphone handelt.

15. System zum Überwachen der physikalischen Eigenschaften eines Fußes eines Patienten nach Anspruch 13, wobei die Rechenvorrichtung von einem Arzt bedient wird.

## Revendications

1. Article chaussant (1) servant à surveiller les propriétés physiques d'un pied diabétique d'un patient comportant une pluralité de capteurs de température (3, 5, 7, 9, 11, 13), chacun servant à mesurer la température de la peau de manière adjacente par rapport au capteur, comprenant un capteur de température (3, 5) situé au niveau d'une région cheville de l'article chaussant, et dans lequel l'un des capteurs (13) comporte un capteur de référence situé à distance des autres capteurs, le capteur de référence (13) étant agencé à des fins d'emplacement sur la jambe inférieure du patient au-dessus de la cheville et espacé par rapport à celle-ci.

2. Article chaussant (1) selon la revendication 1, dans lequel le capteur de référence est monté sur un support séparé physiquement du reste de l'article chaussant.

3. Article chaussant (1) selon la revendication 1 ou la revendication 2, dans lequel le capteur de température situé au niveau d'une région cheville de l'article chaussant est situé au niveau de la malléole ou de manière adjacente par rapport à celle-ci.

4. Article chaussant (1) selon l'une quelconque des revendications précédentes, dans lequel sont prévus un capteur de température situé au niveau d'une région cheville intérieure et un capteur de température situé au niveau d'une région cheville extérieure de l'article chaussant.

5. Article chaussant (1) selon l'une quelconque des revendications précédentes, dans lequel est prévu au moins un capteur de température (7, 9, 11) situé au niveau d'une région semelle de l'article chaussant.

6. Article chaussant (1) selon la revendication 5, dans lequel sont prévus un capteur de température (7) situé au niveau d'une région talon de la semelle de l'article chaussant, un capteur de température (9) situé dans la région d'une 1^{e} région tête du métatarsien de la semelle de l'article chaussant, et un capteur de température (11) situé dans la région d'une 5^{e} région tête du métatarsien de la semelle de l'article chaussant.

7. Article chaussant (1) selon l'une quelconque des revendications précédentes, dans lequel est prévu un capteur de degré d'inclinaison (17) monté sur l'article chaussant.

8. Article chaussant (1) selon l'une quelconque des revendications précédentes, dans lequel est prévu un capteur de pression (15, 65) monté sur l'article chaussant.

9. Article chaussant (1) selon la revendication 8, dans lequel sont prévus une paire de capteurs de pression (65) situés sur la région semelle de l'article chaussant, l'un étant situé de manière adjacente par rapport au talon de l'article chaussant et l'autre étant situé de manière adjacente par rapport à la partie orteils de l'article chaussant.

10. Article chaussant (1) selon l'une quelconque des revendications précédentes, dans lequel est prévu un capteur d'activité (19) servant à détecter quand la chaussure est utilisée sur le pied d'un patient.

11. Article chaussant (1) selon l'une quelconque des revendications précédentes, dans lequel est prévu un capteur de glucose monté sur l'article chaussant.

12. Article chaussant (1) selon l'une quelconque des revendications précédentes, dans lequel le capteur de référence (13) est agencé à des fins d'emplacement sur la jambe inférieure du patient vers le haut le long de la jambe à plus de 0,20 mètres au-dessus de la plante du pied du patient.

13. Système servant à surveiller les propriétés physiques d'un pied d'un patient comportant :
un article chaussant (1) selon la revendication 1, et dans lequel l'article chaussant comporte un microprocesseur servant à collationner des données en provenance de chacun des capteurs et un module de communication servant à transmettre les données collationnées à une station de surveillance à distance ; et
un dispositif informatique à distance exécutant un code de programme d'ordinateur en mesure de recevoir des données en provenance du module de communication et d'afficher les données sur une interface d'utilisateur du dispositif informatique.

14. Système servant à surveiller les propriétés physiques d'un pied d'un patient selon la revendication 13, dans lequel le dispositif informatique est un téléphone intelligent commandé par un patient.

15. Système servant à surveiller les propriétés physiques d'un pied d'un patient selon la revendication 13, dans lequel le dispositif informatique est commandé par un médecin.
